# EUROPEAN PATENT SPECIFICATION

(11) **EP 1 065 302 B1**
(45) Date of publication and mention of the grant of the patent: **13.12.2006**
(21) Application number: 99961427.4
(22) Date of filing: 27.12.1999
(51) Int. Cl.: D01F 6/00

(54) **WETNESS-RESPONSIVE FIBER, PROCESS FOR PRODUCING THE SAME, NONWOVEN FABRIC, AND USE OF THESE**
AUF FEUCHTIGKEIT ANSPRECHENDE FASER, HERSTELLUNGSVERFAHREN, VLIESSTOFFE UND VERWENDUNG
FIBRE REAGISSANT A L'HUMIDITE, PROCEDE DE PRODUCTION DE CETTE FIBRE, NON-TISSE ET UTILISATION DE CETTE FIBRE ET DE CE NON-TISSE

(30) Priority: 28.12.1998 JP 37443798; 28.12.1998 JP 37443898; 12.11.1999 JP 32315499
(43) Date of publication of application: 03.01.2001
(73) Proprietor: Pigeon Corporation, Chiyoda-ku, Tokyo 101-8567 (JP)
(72) Inventor: OMURA, Isao Pigeon Corporation, Chiyoda-ku, Tokyo 101-8567 (JP); INOUE, Osamu Toho Rayon Tokushima Co., Ltd., Itano-gun, Tokushima 771-0286 (JP); KOZEKI, Tomoki Toho Rayon Tokushima Co., Ltd., Itano-gun, Tokushima 771-0286 (JP)
(74) Representative: Matthews, Derek Peter
(86) International application number: PCT/JP1999/007346
(87) International publication number: WO 2000/039373

(56) References cited:
- EP-A- 0 824 105
- EP-A1- 0 071 086
- JP-A- 3 054 234
- US-A- 4 902 792
- US-A- 5 166 231

## Description

### TECHNICAL FIELD

The present invention relates to wet-responsive fibers which can be manufactured into nonwoven fabrics and which can be decomposed into fibers in excess water, preferably under application of external stress such as stirring, and thereby dispersed in an aqueous medium. The invention also relates to a process for producing the wet-responsive fibers, nonwoven fabrics manufactured by the use of the fibers, and use application of the nonwoven fabrics such as body fluid absorbers using the nonwoven fabrics.

### BACKGROUND ART

Cloths have heretofore been used for nursing care goods, baby goods, sanitary napkins, diapers, cleaning cloths, etc. (sometimes referred to as "sanitary goods" en bloc hereinafter). In recent years, however, papers and nonwoven fabrics have been increasingly used instead of cloths. The sanitary goods made of papers or nonwoven fabrics are disposable and very convenient, so that demand for them is expected to increase more and more.

Such sanitary goods need to well absorb aqueous fluid such as urine, and hence papers and nonwoven fabrics used for the sanitary goods need to retain their shape even after they absorb the fluid. On this account, the sanitary goods are formed from papers or nonwoven fabrics having water resistance. Hence, the sanitary goods are insoluble in water, and consequently they cannot be flushed with flush toilet after use and they are disposed of as general refuse.

The sanitary goods once used, however, are forced to bear waste, and it is desirable to dispose of them as rapidly as possible after use. For the disposal of the used sanitary goods, it would be very advantageous if they could be flushed with flush toilet.

The sanitary goods, however, need to have water resistance when they are used, as described above, and as a matter of course, the used sanitary goods also have high water resistance. The sanitary goods having such high water resistance cannot be disposed of by flushing them with flush toilet. For the sanitary goods, the water resistance that is necessary when they are used and decomposability into fibers (water decomposability) desired after they are used are properties conflicting with each other, and it is said that production of sanitary goods having both properties is very difficult.

In contrast, Japanese Patent Laid-Open Publication No. 216889/1992 discloses water-disintegrable nonwoven fabric and binder both of which are hardly dissolved in clean water and body fluids but are apt to be dissolved in sewage.

In this publication, a binder having the following composition is specifically disclosed.

That is, disclosed is a binder which is a copolymer comprising an ethylenic unsaturated carboxylic acid or its anhydride, a crosslinkable monomer and a (meth)acrylic alkyl ester as essential components and having an average molecular weight of 5000 to 10000 and the carboxyl groups thereof are neutralized with monovalent alkali.

The crosslinkable unsaturated monomer is described to be N-methylol(meth)acrylamide or an ether compound thereof.

However, since the carboxyl group is neutralized with monovalent alkali, this monovalent alkali component is dissociated when the nonwoven fabric bears water, and the thus dissociated monovalent alkali component is irritant to the skin. In order to make the nonwoven fabric disintegrable by sewage, the resulting crosslinked structure and the quantity thereof become very important elements when a salt of the above polymer is used, and it is extremely difficult to form crosslinked structure so as to control the solubility of the resin.

EP-A-824105 discloses cellulose particles and refers generally to the presence of cationic and/or anionic groups.

### DISCLOSURE OF THE INVENTION

It is an object of the present invention to provide a wet-responsive fiber and to form therefrom a nonwoven fabric which retains sufficient tensile strength when it is in a wet state and which is decomposed into fibers in a stream of water to be dissolved or dispersed in water.

It is a still further object of the invention to provide a body fluid absorber having an absorbing layer obtained by the use of a nonwoven fabric having the above properties.

it is a still further object of the invention to provide a wet tissue using a nonwoven fabric having the above properties.

These and other objects are provided by a nonwoven fabric comprising wet-responsive fibers as defined below, and by body fluid absorbers and wet tissues formed from said fibers.

Further, the wet-responsive fiber of the invention has a monofilament formed from a resin composition comprising a resin having an anionic group and another resin having a cationic group.

In this invention, the resin having an anionic group is contained in an amount of 1 to 80 % by weight, and the resin having a cationic group is contained in an amount of 1 to 80 % by weight.

The wet-responsive fiber of the invention may also have a monofilament formed from a resin composition comprising a nonionic resin as a base material, a resin having an anionic group and a resin having a cationic group.

In the wet-responsive fiber of the invention, the base resin is contained in an amount of 20 to 95 % by weight, the resin having an anionic group is contained in an amount of 1 to 79 % by weight, and the resin having a cationic group is preferably contained in an amount of 1 to 79 % by weight.

The base resin is preferably viscose rayon, polynosic rayon, cupra or saponified acetate.

The resin having an anionic group is preferably polyacrylic acid salt, carboxymethyl cellulose, carboxymethyl starch, alginic acid, xanthane gum or polymethacrylic acid salt.

The resin having a cationic group is preferably cationized cellulose, cationized starch, cationized cyamoposis gum, cationized dextrin or poly(dimethylmethylenepiperidinium chloride).

Accordingly, independent of the presence or absence of the base resin in the monofilament, a nonwoven fabric can be formed without using a special binder whose monovalent alkali component is dissociated when the fabric holds water and which has been used in the conventional process for forming a nonwoven fabric.

The nonwoven fabric according to the invention comprises fibers formed from a resin composition comprising a cationic resin and an anionic resin.

Particularly, the nonwoven fabric of the invention preferably comprises fibers formed from a resin composition comprising a regenerated cellulose, a cationic resin and an anionic resin.

The nonwoven fabric of the invention more preferably comprises:
fibers formed from a resin composition comprising a regenerated cellulose, a cationic resin and an anionic resin, and
a water-indecomposable short fiber.

When the nonwoven fabric of the invention comprises fibers containing the regenerated cellulose, the regenerated cellulose is contained in an amount of 20 to 98 % by weight, the cationic resin is contained in an amount of 1 to 79 % by weight, and the anionic resin is contained in an amount of 1 to 79 % by weight.

When the nonwoven fabric of the invention comprises fibers containing regenerated cellulose, the regenerated cellulose is preferably viscose rayon, polynosic rayon, cupra or saponified acetate.

The cationic resin is preferably cationized cellulose, cationized starch, cationized cyamoposis gum, cationized dextrin or poly(dimethylmethylenepiperidinium chloride).

The anionic resin is preferably polyacrylic acid salt, carboxymethyl cellulose, carboxymethyl starch, alginic acid, xanthane gum or polymethacrylic acid salt.

The cationic resin and the anionic resin for use in the invention are water-soluble polymers. From the water-soluble polymers and regenerated cellulose, a resin composition is prepared. From the resin composition, fibers are shaped, and by the use of the fibers, a nonwoven fabric is fabricated. Therefore, the nonwoven fabric can be imparted with water decomposability even if a special binder whose monovalent alkali component is dissociated when the binder contains water and which has been used in the conventional process for forming nonwoven fabrics is not used. The nonwoven fabric of the invention retains sufficient tensile strength when it is in a wet state, but the nonwoven fabric is decomposed into fibers and dissolved or dispersed in water when it is brought into contact with a large amount of water by, for example, exposing it to a stream of water.

The body fluid absorber according to the invention has an absorbing layer comprising at least one nonwoven fabric selected from the group consisting of:
(a) a nonwoven fabric comprising fibers formed from a resin composition comprising a cationic resin and an anionic resin,
(b) a nonwoven fabric comprising fibers formed from a resin composition comprising a regenerated cellulose, a cationic resin and an anionic resin, and
(c) a nonwoven fabric comprising fibers formed from a resin composition comprising a regenerated cellulose, a cationic resin and an anionic resin, and a water-indecomposable short fiber.

The nonwoven fabric retains sufficient tensile strength even when it is in a wet state, and the nonwoven fabric is decomposed into fibers by a stream of water or the like. Therefore, by constituting the body fluid absorber of the invention using the nonwoven fabric, the body fluid absorber can be easily disposed of after use. Accordingly, the body fluid absorber of the invention can be applied to various sanitary goods.

The wet tissue according to the invention comprises:
at least one nonwoven fabric selected from the group consisting of:
   (a) a nonwoven fabric comprising fibers formed from a resin composition comprising a cationic resin and an anionic resin,
   (b) a nonwoven fabric comprising fibers formed from a resin composition comprising a regenerated cellulose, a cationic resin and an anionic resin, and
   (c) a nonwoven fabric comprising fibers formed from a resin composition comprising a regenerated cellulose, a cationic resin and an anionic resin, and a water-indecomposable short fiber; and
a liquid agent with which the nonwoven fabric is impregnated.

The wet tissue has sufficient tensile strength though it is impregnated with a liquid agent (in a wet state). Moreover, the wet tissue is decomposed into fibers by the contact with a large amount of water, and therefore the wet tissue can be easily disposed of after use.

### BRIEF DESCRIPTION OF THE DRAWINGS

Figs. 1 are diagrams illustrating an embodiment of a wet tissue package kit, and wherein (a) shows a lid section, (b) shows a packaged wet tissue package and (c) shows a container body.
Fig. 2 is a sectional view illustrating an embodiment of a manner to fold wet tissues.
Figs. 3 are explanatory diagrams illustrating a wet tissue web having perforations.
Fig. 4 is a sectional view illustrating the kit in use.
Fig. 5 is a perspective view illustrating the kit in use.
Fig. 6 is an explanatory view illustrating another embodiment of the kit.
Fig. 7 is a plan view illustrating another embodiment of a resistive barrier plate.
Fig. 8 is a perspective view illustrating another embodiment of a takeout opening of the wet tissue package.
Fig. 9 is a detailed plan view of the resistive barrier plate shown in Fig. 1.
Figs. 10 are plan views illustrating various embodiments of the resistive barrier plates.

### BEST MODE FOR CARRYING OUT THE INVENTION

The wet-responsive fiber of the invention, the process for producing the wet-responsive fiber, the nonwoven fabric formed by the use of the fiber, the body fluid absorber and the wet tissue are described in detail hereinafter.

First, the wet-responsive fiber of the invention is described.

The wet-responsive fiber of the invention is:
(A) a fiber having a monofilament formed from a resin composition of a water-soluble polymer comprising a resin having an anionic group and another resin having a cationic group, or
(B) a fiber having a monofilament formed from a resin composition comprising the water-soluble polymer (i.e., water-soluble polymer comprising a resin having an anionic group and another resin having a cationic group) and a resin as a base.

The "water-soluble polymer" means a substance that can be decomposed when brought into contact with water.

The former fiber (A) and the latter fiber (B) both have a monofilament containing the water-soluble polymer, so that they have properties of "response to wet" that their characteristics are changed when they are brought into contact with water.

In order that the former fiber (A) may retain sufficient strength when it is in a wet state and may be dissolved or dispersed when it contains excess water, the resin having an anionic group is contained in this fiber in an amount of 1 to 80 % by weight, and the resin having a cationic group is contained in this fiber in an amount of 1 to 80 % by weight.

In the latter fiber (B), the base resin is contained in an amount of 20 to 95 % by weight, preferably 30 to 85 % by weight, more preferably 50 to 80 % by weight, particularly preferably 70 to 80 % by weight, the anionic resin is contained in an amount of 1 to 79 % by weight, preferably 5 to 70 % by weight, more preferably 10 to 50 % by weight, particularly preferably 15 to 30 % by weight, and the cationic resin is contained in an amount of 1 to 79 % by weight, preferably 5 to 70 % by weight, more preferably 10 to 50 % by weight, particularly preferably 15 to 30 % by weight, each amount being based on 100 % by weight of the total of all components.

The wet-responsive fiber of the invention may contain further ingredient(s) in addition to the above components without departing from the objects and the effects of the present invention. In this case, the content of the additional ingredient(s) is not more than 80 % by weight, preferably not more than 40 % by weight, particularly preferably not more than 10 % by weight.

The fiber containing those components in the above amounts has excellent wet response. That is, the fiber is well-balanced between retention of the tensile strength in a wet state and appearance of the water decomposability.

The fiber can be produced by, for example, the following process.

The wet-responsive fiber (A) can be produced as follows. A 50% alcohol solution (e.g., ethanol solution) containing about 1 % by weight of polyacrylic acid is mixed with a 50% alcohol solution (e.g., ethanol solution) containing about 1 % by weight of cationized cellulose. The mixture is extruded through a spinning nozzle, followed by drying.

The wet-responsive fiber (B) can be produced as follows. Pulp is immersed in an alkali aqueous solution such as a sodium hydroxide solution to alter the pulp to alkali cellulose, and the alkali cellulose is aged. Then, a sulfide such as carbon disulfide is added to lead the alkali cellulose into sulfidizing so as to give sodium cellulose xanthate, and the sodium cellulose xanthate is dissolved in a medium to prepare a spinning solution. To the spinning solution, an aqueous solution of cationized cellulose and an aqueous solution of sodium polyacrylate are added, and they are mixed. The mixture thus obtained is then extruded into a solidifying medium through a spinning nozzle to be spun.

The length of the wet-responsive fiber is usually not less than 2 mm, preferably 20 to 100 mm, more preferably 30 to 80 mm.

Examples of the resins having an anionic group for constituting the wet-responsive fibers (A) and (B) include polyacrylic acid salt, carboxymethyl cellulose, carboxymethyl starch, polyalginic acid, xanthane gum and polymethacrylic acid salt. Of these, polyacrylic acid salt, carboxymethyl cellulose and carboxymethyl starch are preferable from the viewpoints of hygienic qualities, safety and water decomposability.

Examples of the resins having a cationic group include cationized cellulose, cationized starch, cationized cyamoposis gum, cationized dextrin and poly(dimethylmethylenepiperidinium chloride). Of these, cationized cellulose and cationized starch are preferable from the viewpoints of hygienic qualities, safety and water decomposability.

Examples of the nonionic resins as the base material for the wet-responsive fiber (B) include viscose rayon, polynosic rayon, cupra and saponified acetate. Of these, viscose rayon, polynosic rayon and cupra are preferable from the viewpoints of hygienic qualities and safety.

Examples of the additional ingredients which can be contained in the wet-sensitive fibers (A) and (B) include pulp, cotton, hemp (ramie) and polyester fibers.

The wet-responsive fiber is favorably used for fabricating a nonwoven fabric having water decomposability.

To prepare the nonwoven fabric from the fibers, the fibers are entangled or bonded to each other so that the water decomposability of the invention may appear. For example, wet process, wet spun lacing, dry spun lacing, needle punching, chemical bonding and thermal bonding are available.

The nonwoven fabric thus obtained retains sufficient strength when it is in a wet state, and exhibits water decomposability when it contains excess water. Moreover, the nonwoven fabric can be formed without using the conventional binder whose monovalent alkali moiety is dissociated when wet and which has been heretofore used, so that no skin irritation takes place even when the nonwoven fabric is brought into close contact with the skin. Accordingly, the nonwoven fabric can be applied to an absorbing layer of a sanitary good such as a sanitary napkin.

Now, the nonwoven fabric is described below in more detail.

The nonwoven fabric of the invention usually has water decomposability. In the present invention, the term "water decomposability" means that the nonwoven fabric is decomposed into fibers and dispersed in water when it contains a certain amount or more of water.

The nonwoven fabric of the invention comprises fibers formed from a resin composition comprising a cationic resin and an anionic resin. Usually, the resin composition further contains a base resin. Examples of the resins used as the base of the composition for the nonwoven fabric of the invention include the aforesaid viscose rayon, polynosic rayon, cupra and saponified acetate, and polyolefin, polyester, polyamide, cellulose and regenerated cellulose. These resins can be used singly or in combination. In the resin composition for forming the fibers, the base resin is contained in an amount of usually 1 to 99 % by weight, preferably 10 to 95 % by weight, based on 100 % by weight of the resin composition. In the present invention, it is particularly preferable to use regenerated cellulose as the base resin.

Accordingly, the nonwoven fabric preferably used in the invention comprises fibers formed from a resin composition comprising the regenerated cellulose, the cationic resin and the anionic resin. The preferred nonwoven fabric is described below in more detail.

The fibers for constituting the nonwoven fabric of the invention are obtained from a resin composition comprising the regenerated cellulose as the base resin and the later-described water-soluble polymer. The "water-soluble polymer" means a substance having properties that it swells or is dissolved by the contact with water.

By forming a nonwoven fabric using fibers comprising the water-soluble polymer and regenerated cellulose, the nonwoven fabric comes to have "stimulus-responsive water decomposability (wet-responsive water decomposability)", even though it has no specific binder which has been used conventionally in this art, that water decomposability appears when the fabric receives a stimulus, for example, it is stirred in water.

In the nonwoven fabric having the stimulus-responsive water decomposability, the regenerated cellulose is contained in an amount of usually 20 to 98 % by weight, preferably 20 to 95 % by weight, particularly preferably 30 to 85 % by weight, more preferably 50 to 80 % by weight, still more preferably 70 to 80 % by weight, and the water-soluble polymer is contained in an amount of usually 2 to 80 % by weight, preferably 5 to 80 % by weight, particularly preferably 15 to 70 % by weight, more preferably 20 to 50 % by weight, still more preferably 20 to 30 % by weight, each amount being based on 100 % by weight of the total of the resin components for forming the fibers of the nonwoven fabric.

Particularly with respect to the water-soluble polymer, the cationic resin is contained in an amount of usually 1 to 79 % by weight, preferably 2 to 68 % by weight, particularly preferably 2 to 50 % by weight, and the anionic resin is contained in an amount of usually 1 to 79 % by weight, preferably 2 to 68 % by weight, particularly preferably 13 to 48 % by weight.

When the amounts of the regenerated cellulose and the water-soluble polymer (i.e., cationic resin and anionic resin) are in the above ranges, the response to stimulus is good. That is, the fabric is well-balanced between retention of the tensile strength in a wet state and appearance of the water decomposability when coming into contact with an appropriate amount of water.

Further, when their amounts are in the above ranges, the water-soluble polymer can be prevented from falling off, and therefore sticking of the fibers hardly occurs. Hence, the same facilities to manufacture the fibers and the nonwoven fabric as the conventional ones can be used, resulting in industrial advantages. Moreover, the yield can be increased, resulting in a merit in the production cost.

The resin composition for forming the fibers may contain additional ingredient(s) other than the base resin (preferably regenerated cellulose) and the water-soluble polymer without departing from the objects and the effects of the present invention. The content of the additional ingredient(s) is not more than 90 % by weight, preferably not more than 40 % by weight, particularly preferably not more than 10 % by weight.

In the manufacture of the nonwoven fabric of the invention, fibers are first produced by the above process, and then the nonwoven fabric is manufactured using the obtained fibers.

That is, pulp is immersed in a sodium hydroxide solution to change the pulp into alkali cellulose, and the alkali cellulose is aged. Then, carbon disulfide is added to lead the alkali cellulose into sulfidizing so as to give sodium cellulose xanthate. The sodium cellulose xanthate is dissolved in the medium to prepare a spinning solution. To the spinning solution, an aqueous solution of cationized cellulose and an aqueous solution of sodium polyacrylate are added, and they are mixed. The mixture is then extruded into a solidifying medium through a spinning nozzle to be spun.

The length of the fibers is usually not less than 2 mm, preferably 20 to 100 mm, more preferably 30 to 80 mm.

The fibers are entangled or bonded each other so that the water decomposability may be retained, whereby the nonwoven fabric is manufactured. Specifically, wet process, wet spun lacing, dry spun lacing, needle punching, chemical bonding and thermal bonding are available. Use of a chemical binder, however, is not favorable in view of hygienic qualities, as described above. In the present invention, the most preferable process to manufacture the nonwoven fabric is wet spun lacing or needle punching.

Examples of the regenerated cellulose include viscose rayon, polynosic rayon, cupra and saponified acetate. Of these, viscose rayon, polynosic rayon and cupra are preferable from the viewpoints of hygienic qualities and safety. The regenerated cellulose can be used singly or in combination.

Examples of the cationic resins include cationized cellulose, cationized starch, cationized cyamoposis gum, cationized dextrin and poly(dimethylmethylenepiperidinium chloride). Of these, cationized cellulose and cationized starch are preferable from the viewpoints of hygienic qualities, safety and water decomposability. These cationic resins can be used singly or in combination.

Examples of the anionic resins include polyacrylic acid salt, carboxymethyl cellulose, carboxymethyl starch, polyalginic acid, xanthane gum and polymethacrylic acid salt. Of these, polyacrylic acid salt, carboxymethyl cellulose and carboxymethyl starch are preferable from the viewpoints of hygienic qualities, safety and water decomposability. These anionic resins can be used singly or in combination.

Examples of the other ingredients used in the manufacture of the nonwoven fabric include pulp, rayon, polypropylene fusion bonded fibers, cotton, hemp (ramie) and polyester fibers.

The water-decomposable fibers for manufacturing the nonwoven fabric of the invention are preferably used in the form of a web.

The nonwoven fabric of the invention can be manufactured by first producing fibers (water-decomposable fibers or wet-responsive fibers) from a resin composition preferably comprising the regenerated cellulose, the cationic resin and the anionic resins as described above and then subjecting the fibers to, for example, the above process. In the manufacture of the nonwoven fabric, to the fibers may be further added water-indecomposable short fibers.

That is, the preferred nonwoven fabric of the invention comprises:
fibers formed from a resin composition comprising the regenerated cellulose, the cationic resin and the anionic resin, and
the water-indecomposable short fibers.

The water-indecomposable short fibers are resin short fibers not having properties that they are dissolved in water or stably dispersed in water. Examples of the short fibers include synthetic fibers, natural fibers and regenerated fibers, such as pulp fibers, regenerated cellulose fibers (e.g., viscose rayon, polynosic rayon, cupra, saponified acetate), polyamide fibers, polyester fibers, polyacrylic fibers, polyurethane fibers and polyolefin fibers. Of the above water-indecomposable short fibers, the regenerated cellulose fibers are preferably employed. By the use of the regenerated cellulose fibers, the resulting nonwoven fabric is remarkably improved in hand and feeling, and besides the water absorption properties of the nonwoven fabric is enhanced. The water-indecomposable short fibers used herein have such fiber lengths that complicated entanglement which impairs water decomposability of the nonwoven fabric of the invention does not occur. The average fiber length of the water-indecomposable short fibers is usually less than 80 mm, preferably less than 40 mm, more preferably less than 20 mm. In order that the nonwoven fabric manufactured from a mixture of the water-decomposable fibers and the water-indecomposable fibers may keep favorable water decomposability, the average fiber length of the water-indecomposable short fibers is desired to be short. The lower limit of the average fiber length of the water-indecomposable short fibers has only to be such a length as the water-indecomposable short fibers do not flow out during the manufacture of the nonwoven fabric. The lower limit of the average fiber length is usually 0.1 mm, preferably about 0.5 mm, though it varies depending upon the process for the manufacture of the nonwoven fabric. The water-indecomposable fibers are desired to be used in the form of a web, similarly to the water-decomposable fibers.

In the nonwoven fabric, the water-decomposable fibers and the water-indecomposable fibers are used in a mixing ratio of usually 1:99 to 99:1, preferably 30:70 to 70:30. That is , the nonwoven fabric can be manufactured by mixing the water-decomposable fibers with the water-indecomposable fibers in the above ratio and subjecting the mixture to a conventional process, such as wet process, wet spun lacing, dry spun lacing, needle punching, chemical bonding or thermal bonding, preferably dry spun lacing or needle punching.

Although the nonwoven fabric mentioned above contains water-indecomposable fibers, it has water decomposability.

The METSUKE, i.e., weight per unit area, of the nonwoven fabric thus obtained is in the range of usually 20 to 60 g/m², and the thickness thereof is in the range of usually 0.1 to 0.6 mm. The nonwoven fabric retains sufficient strength when it is in a wet state, and exhibits water decomposability when it is stirred in excess water. The nonwoven fabric of the invention exhibits water decomposability when contacted with a large amount of water, and especially in an alkaline aqueous solution, it exhibits remarkably excellent water decomposability. Further, since the nonwoven fabric is manufactured without using a binder whose monovalent alkali moiety is dissociated when the fabric contains water and which has been heretofore used, irritation of the skin is hardly brought about even when the nonwoven fabric is directly contacted with the skin. Accordingly, the nonwoven fabric can be applied to an absorbing layer of sanitary good such as paper diaper, urine absorbing pad or sanitary napkin.

The body fluid absorber of the invention uses the nonwoven fabric as its absorbing layer. This body fluid absorber has an absorbing layer comprising at least one nonwoven fabric selected from the group consisting of:
(a) a nonwoven fabric comprising fibers formed from a resin composition comprising a cationic resin and an anionic resin,
(b) a nonwoven fabric comprising fibers formed from a resin composition comprising a regenerated cellulose, a cationic resin and an anionic resin, and
(c) a nonwoven fabric comprising fibers formed from a resin composition comprising a regenerated cellulose, a cationic resin and an anionic resin, and water-indecomposable short fibers.

The body fluid absorber has an absorbing layer of the nonwoven fabric of the invention which is usually disintegrated by the contact with a large amount of water as described above. In the body fluid absorber, it is preferable to form a water-impermeable layer (back sheet) as the outermost layer so that the absorbed water does not ooze out, and a surface layer may be provided in order to prevent returning of the absorbed water. In the absorbing layer, a highly water-absorbing material such as a polymer absorber may be added in an amount not detrimental to the water decomposability of the nonwoven fabric. The body fluid absorber can be disposed of after use by separating the water-absorbing layer of the nonwoven fabric having water decomposability from the water-indecomposable back sheet and then exposing the thus separated absorbing layer to water to be disintegrated.

In the body fluid absorber of the invention, the absorbing layer is formed from the nonwoven fabric that is manufactured without using a binder containing an alkali moiety, so that dissociation of a monovalent alkali component does not take place when the nonwoven fabric contains water. Hence, even if the absorbing layer is brought into direct contact with the skin in the use of the absorber, there is no fear of irritation of the skin caused by the alkali moiety.

The absorbing layer retains tensile strength of a certain level when it absorbs excretions, secretions, etc., and since the nonwoven fabric constituting the absorbing layer has the aforesaid stimulus-responsive water decomposability, the absorbing layer is decomposed into fibers and dispersed in water when the layer is stimulated by exposing it to a stream of water or the like. On this account, immediately after the body fluid absorber is used, the absorbing layer can be separated and directly flushed with flush toilet. Thus, the body fluid absorber can be easily and rapidly disposed of after use.

The wet tissue of the invention comprises:
at least one nonwoven fabric selected from the group consisting of:
   (a) a nonwoven fabric comprising fibers formed from a resin composition comprising a cationic resin and an anionic resin,
   (b) a nonwoven fabric comprising fibers formed from a resin composition comprising a regenerated cellulose, a cationic resin and an anionic resin, and
   (c) a nonwoven fabric comprising fibers formed from a resin composition comprising a regenerated cellulose, a cationic resin and an anionic resin, and water-indecomposable short fibers; and
a liquid agent with which the nonwoven fabric is impregnated.

The wet tissue utilizes properties that the nonwoven fabric is hardly impaired in its shape when it is contacted with a small amount of water but it tends to be decomposed by the contact with a large amount of water. This wet tissue can be produced by impregnating the nonwoven fabric with a liquid agent in an amount of usually 1 to 5 times (by weight) as much as the nonwoven fabric. Examples of the liquid agents used herein include water and water-alcohol mixture. To the liquid agent, antiseptics (e.g., aromatic antiseptics such as sodium benzoate), moisture-keeping agents (e.g., polyalcohols such as ethylene glycol, propylene glycol and glycerin), surface active agents, perfumes, etc. can be added.

The wet tissue can be supplied by being encased in a water-impermeable packaging bag. The wet tissue of the invention is preferably used as a stationary wet tissue supply kit comprising a container capable of retaining its own shape and a wet tissue package in which the nonwoven fabric impregnated with the liquid agent is encased in a packaging bag.

The stationary wet tissue supply kit is described below in more detail.

In this stationary wet tissue supply kit,
(i) the container comprises a storage space member having a capacity for the packaging bag of the wet tissue, a lid member that is lifted or shut down when the packaging bag is replaced in the storage space, an opening section capable of being opened or closed and to take the wet tissues out of the container one by one, and a resistive barrier plate which is located between the storage space and the opening section and which has a takeout aperture that becomes a resister to operations of taking out the wet tissues, and
(ii) the wet tissue package comprises wet tissues each of which are folded and superposed on another so as to have an overlapped portion with the another and encased in a packaging bag having a wet tissue takeout opening.

In the kit, the wet tissue package (ii) may be replaced with a wet tissue package comprising a web wherein each strips of wet tissues are continuously aligned via perforations and is encased in a packaging bag having a wet tissue takeout opening.

A sealing member adhesion bonded to the takeout opening of the packaging bag containing the wet tissues is peeled off, and the packaging bag containing the wet tissues is encased as it is in the container body of the wet tissue supply kit. The wet tissues can be exhaustively taken out one by one with their unfolded form from the opening section of the closed container.

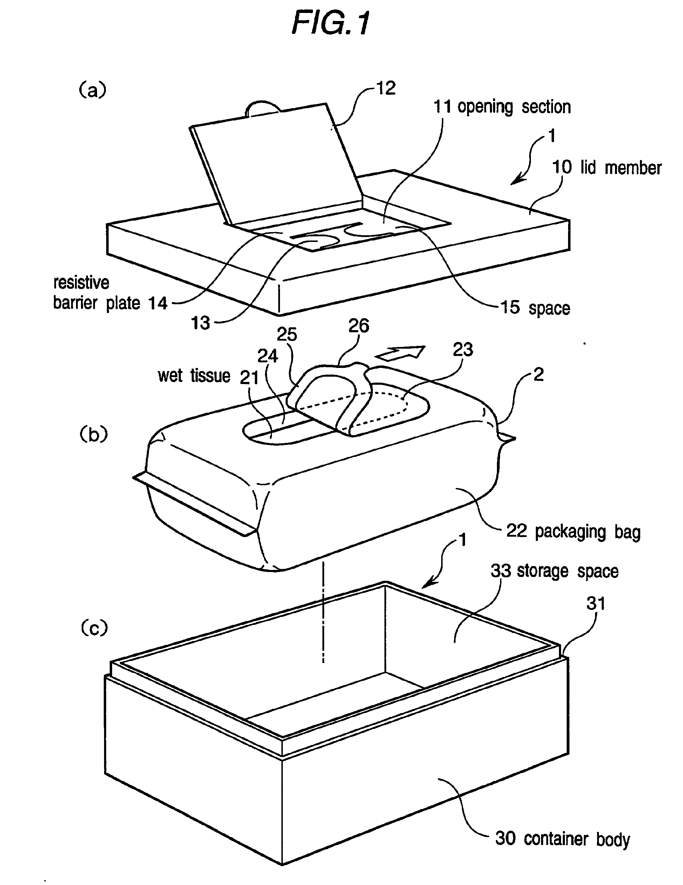

Fig. 1 illustrates an embodiment of the kit. Fig. 1(a) is an explanatory view illustrating a lid member of a container of a kit using the wet tissue of the invention, Fig. 1(b) is an explanatory view illustrating a wet tissue package, and Fig. 1(c) is an explanatory view illustrating a container body. As shown in Fig. 1, the kit using the wet tissue of the invention comprises a container 1 capable of being closed and a wet tissue package 2 comprising the nonwoven fabrics having been impregnated with a liquid agent and encased in a water-impermeable packaging bag.

### (i) Container

The container 1 for constituting the kit is in a shape of an almost rectangular parallelepiped as a whole and is made from such a material as the container can retain its own shape. As the material, a molding material that is relatively rigid and has somewhat flexibility, e.g., a synthetic resin such as polyethylene or polypropylene, is preferably employed. Other than the synthetic resin, a metallic plate or a paper may be used as the material of the container. The container has only to retain its own shape, and the container may be one obtained by internally or externally reinforcing a flexible sheet so that the shape can be retained.

The container 1 includes a container body 30 and a lid member 10 which can be closely fitted to the container body. The container body 30 is provided at the top edge with a receiving portion 31 having a step on which the lid member 10 is mounted, and embraces a storage space 33 to place therein the wet tissue package 2.

In almost the center of the lid member 10, there is provided a rectangular opening section 11 through which the wet tissue is taken out. In this opening section, an open-close lid 12 is fitted at its one side so that the lid becomes turnable. The open-close lid 12 is so closely provided that a liquid or a gas does not leak from the opening section 11.

At the area corresponding to the opening section 11 inside the lid member 10 is provided a resistive barrier plate 14 having a takeout aperture with U-shaped elongation lips 13, and by the resistive barrier plate, a wet tissue to be taken out next is held. Between the resistive barrier plate 14 and the opening section 11 on the upper part of the lid member 10, a space 15 is formed, and in this space, a tip of the wet tissue held by the resistive barrier plate stays. The resistive barrier plate is arranged so that it may serve as a resister to the operation of taking out the wet tissue. Referring to the embodiment, by virtue of the resistance based on the elasticity of the U-shaped elongation lips 13, a wet tissue that has come out together with the previous one is held to the opening section.

### (ii) Wet tissue package

The nonwoven fabric filled in the wet tissue package 2 is one having been impregnated with a liquid agent. As described above, the nonwoven fabric has properties that it is not disintegrated by a small amount of water but is disintegrated when contacted with a large amount of water, preferably under application of external stress such as stirring of water or stream of water. Conventional tissue paper or pocket tissue paper used in a dry state does not have such properties. That is, the conventional tissue paper used in a dry state is markedly lowered in the tensile strength when wetted, and is disintegrated within several hours after contacted with a liquid, so that each piece of the tissue paper cannot be taken out separately.

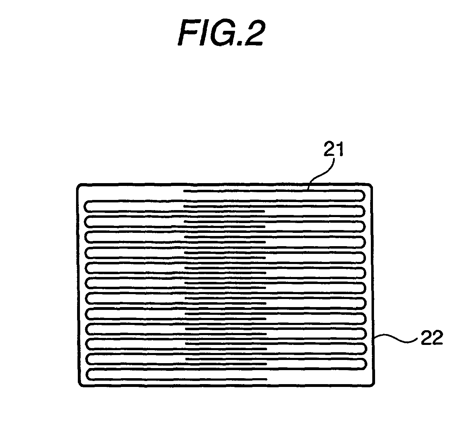

With respect to the wet tissues encased in a packaging bag 22, the each nonwoven fabrics are folded and superposed on another so as to have an overlapped portion with the another. When one piece of tissue paper is taken out from the opening section 11 of the container 1 through the resistive barrier plate, the next one is held by the resistive barrier plate and waits the subsequent use. In order to exhausively take out the wet tissues like this, the overlapped area between the first and the second tissues is not more than 1/2, preferably not more than 1/4, more preferably not more than 1/10, of the area of one wet tissue, differently from the dry type wet tissues. A preferred embodiment of a manner to fold the wet tissues with superposing is shown in Fig. 2.

Similarly to the dry type wet tissues, the overlapped area between the wet tissues of the invention can be set to about 1/2 by controlling and decreasing frictional force between the overlapped wet tissues using various means.

For example, the nonwoven fabric is subjected to creping or embossing to form protrusions and depressions and thereby reduce the contact area between the overlapped wet tissues. Further, the frictional force can be decreased by lowering the water content in the wet tissues.

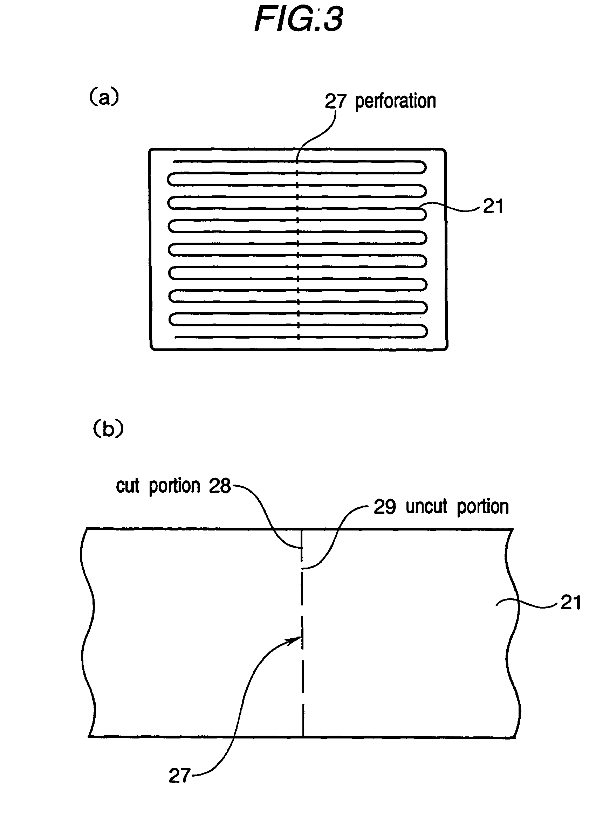

In the present invention, a web of wet tissue 21 having perforations as shown in Figs. 3(a) and 3(b) may be encased in a packaging bag. When the wet tissue corresponding to one strip is taken out from the opening section 11 of the container 1 through the resistive barrier plate, the next strip is held by the resistive barrier plate to tear the web at the perforation 27 and waits the next use. In order to take out sequencially the wet tissue one strip after another by tearing the web at the perforations like this, the length of a cut portion 28 of the perforation 27 is desired to be longer than the length of an uncut portion 29 of the perforation 27 as shown in Fig. 3(b). The material used for the wet tissue has a relatively high tensile strength, so that the force required to separate each strip is adjusted. For example, the ratio of the length of the uncut portion 29 to the length of the cut portion 28 is set to not more than 1/5, preferably not more than 1/20, more preferably about 1/50. '

The packaging bag 22 for the wet tissues is preferably formed from a flexible packaging material (e.g., plastic film or laminated film) which is not permeated by the liquid agent for impregnation of the wet tissues and which prevents invasion of various bacteria. On the upper surface of the packaging bag 22, a takeout opening 24 with a cut 23 having been previously formed by appropriate means such as perforating or half cut is provided so as to easily open the bag prior to use, and a sealing member 26 coated with an adhesive (pressure-sensitive adhesive) 25 on its back surface is adhesion bonded to the bag so as to cover the takeout opening 24. The wet tissue supply kit is a stationary type, and the sealing member 26 is peeled off from the packaging bag before the bag is encased in the container, so that the adhesive applied to the sealing member does not need to have re-adhesion properties (properties that the adhesive is capable of adhering again after once separated).

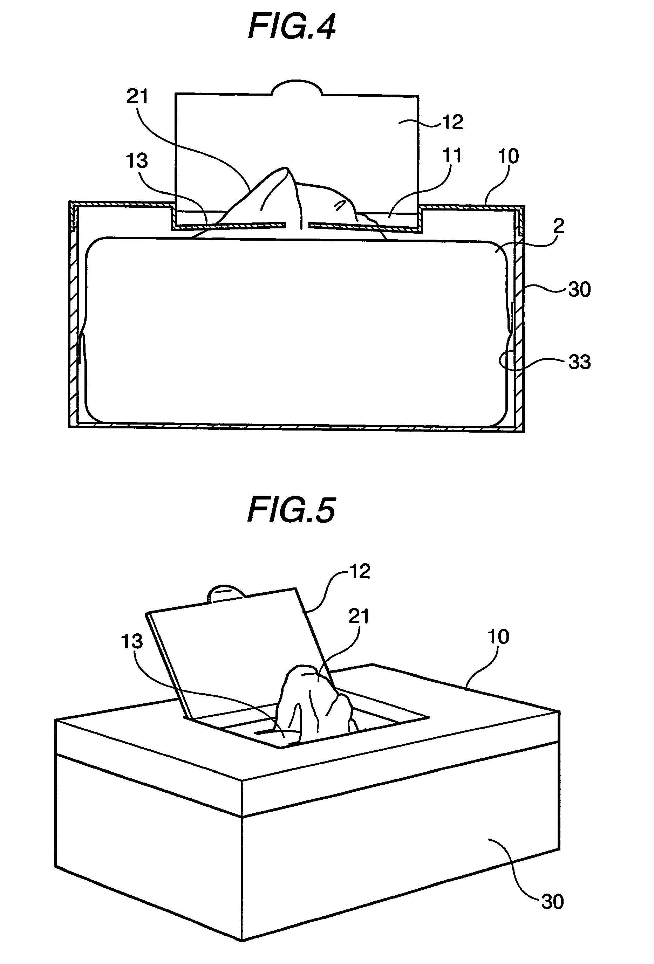

When the sealing member 26 is peeled off prior to use of the wet tissue, a part of a film at the position of the takeout opening 24 having a cut of a closed loop is removed together with the sealing member 26, whereby the packaging bag is opened. As shown in Fig. 4 and Fig. 5, the packaging bag is encased in the container body 30, then a lid member 10 is put on, and the first wet tissue 21 is allowed to pass through the takeout aperture 17 of the resistive barrier plate 14.

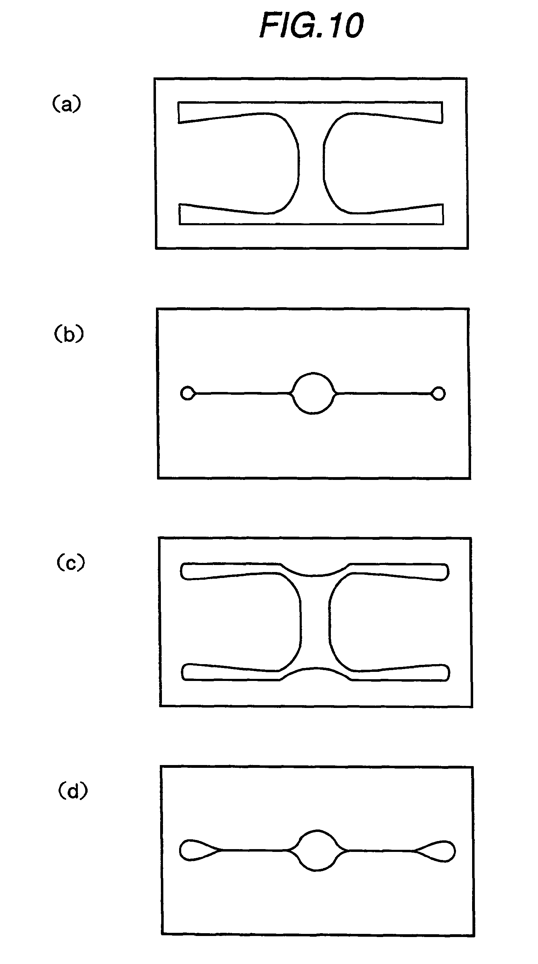

Examples of the shapes of the resistive barrier plates include those shown in Fig. 1, Fig. 7 and Figs. 10(a) to 10(d), and preferable is a resistive barrier plate provided with elongated resistive lips which have moderate flexibility and rigidity by appropriate selection of a material, thickness of a material, shape and the like.

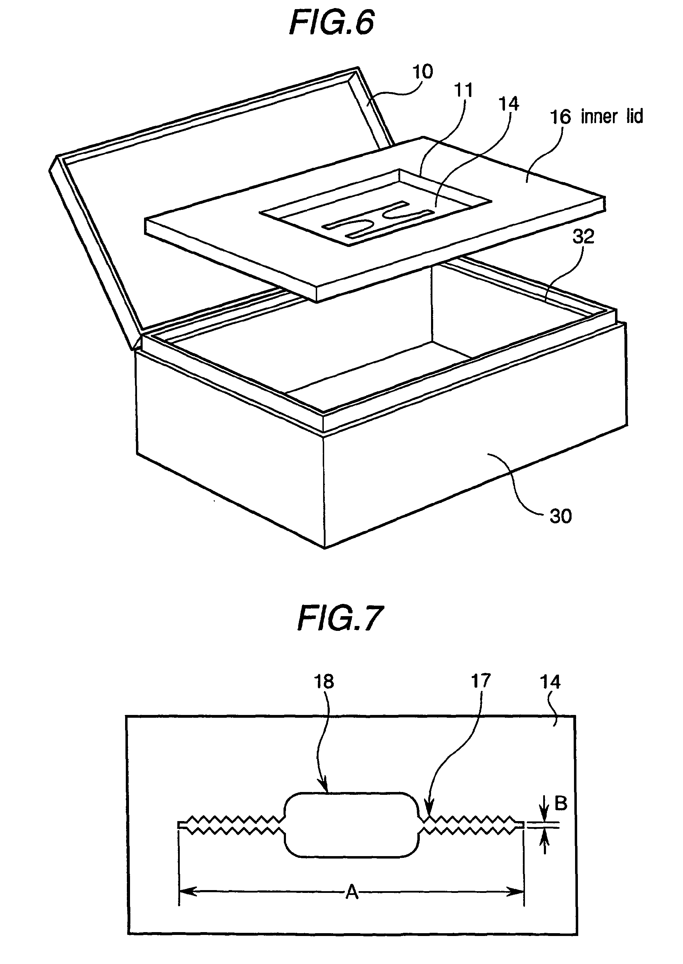

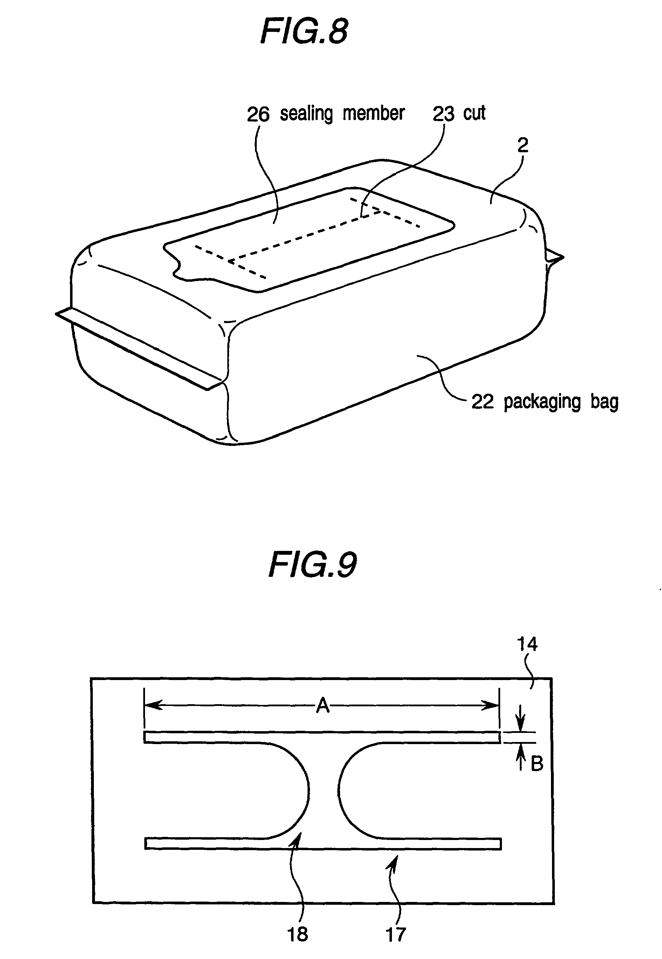

In the resistive barrier plate, the maximum length (A) of the takeout aperture is preferably in the range of 15 to 99 % of the width of the wet tissue that passes through the aperture, as shown in Fig. 7 and Fig. 9. If the maximum length is shorter than the lower limit of the above range, the wet tissue cannot be taken out. If the maximum length is longer than the upper limit of the above range, holding of the wet tissue cannot be made sufficiently, designing of the container is not made conveniently, and there is high possibility of introduction of foreign matters.

On the other hand, the minimum width (B) of the takeout aperture is preferably in the range of 1 mm to 1 cm. If the minimum width is narrower than the lower limit of the above range, the wet tissue cannot be taken out. If the minimum width is wider than the upper limit of the above range, it becomes difficult to hold the wet tissue.

The width of the expanded part 18 located near the center in the direction of the maximum length of the takeout aperture of the resistive barrier plate is preferred to be the maximum width of the takeout aperture 17. By virtue of the enlarged part 18 provided near the center of the takeout aperture, the wet tissue gathered to the takeout aperture having a smaller width (width A) than that of the wet tissue can be relieved, and hence it becomes feasible to smoothly take out the wet tissue with moderately holding the wet tissue.

Fig. 6 is a perspective view illustrating another embodiment of the container of the kit. In this embodiment, the lid member 10 is fitted to the container body 30 so that the lid member can turn to open the container. The resistive barrier plate 14 is provided in an inner lid 16, and the inner lid 16 is closely mounted on an inner lid receiving portion 32 formed inside the container body. In the container of the kit, the lid section for replacing the packaging bag may be provided on the bottom or the side of the container instead of the top of the container shown in Fig. 1 or Fig. 6.

Fig. 8 is a perspective view illustrating another embodiment of the takeout opening of the packaging bag for the wet tissues. In this embodiment, a takeout opening having a laterally long, H-shaped cut 23 is provided. The takeout opening in this shape has a small open area because the sheet remains even after the bag is cut open, and therefore secondary contamination with bacteria in air or vaporization of a liquid component can be prevented.

The stationary wet tissue supply kit mentioned above can be extremely easily supplemented or refilled with wet tissues and is hygienic. Further, due to the stationary type, the necessary number of wet tissues strips can be rapidly taken out one by one in their unfolded form that is convenient in use, even with one hand.

As described above, the nonwoven fabric of the invention can be used for the wet tissue. This nonwoven fabric is not water-decomposed when it is in a packaging bag together with a liquid agent and can be taken out from the container one by one, but the nonwoven fabric is decomposed if once contacted with a large amount of water. Accordingly, the wet tissue can be disposed of by flushing it with flush toilet after use. The wet tissue using the nonwoven fabric of the invention can be applied to sanitary goods, e.g., cleaning goods for babies and nursing care, particularly goods to clean babies' bottoms and toilet cleaning goods.

### EFFECT OF THE INVENTION

According to the present invention, a wet-responsive fiber having good balance between retention of tensile strength in a wet state and appearance of water decomposability can be provided. By the use of the fiber, a nonwoven fabric which retains sufficient tensile strength when it is in a wet state and which is decomposed into fibers and dissolved or dispersed in water can be readily provided.

The nonwoven fabric of the invention retains tensile strength of a certain level in a wet state and is decomposed into fibers and dissolved or dispersed when it is contacted with a large amount of water by, for example, exposing it to a stream of water, though the fabric does not use any binder that is conventionally used for manufacturing nonwoven fabrics.

According to the body fluid absorber of the invention, there is no fear of irritation of the skin even if its absorbing layer is directly put on the skin in the use of the absorber, because any binder whose monovalent alkali moiety is dissociated when it contains water is not used in the manufacture of a nonwoven fabric for forming the absorbing layer.

The absorbing layer retains tensile strength of a certain level when it absorbs humors such as excretions or secretions, but, when the absorbing layer is stimulated by exposing it to a stream of water or the like, this absorbing layer is decomposed into fibers and dispersed in water because the nonwoven fabric has stimulus-responsive water decomposability. On this account, only the absorbing layer can be separated from the body fluid absorber and directly flushed with flush toilet after the body fluid absorber is used. Thus, the body fluid absorber of the invention can be easily and rapidly disposed of after use.

The wet tissue of the invention comprises the above-mentioned nonwoven fabric and a liquid agent. When the wet tissue impregnated with the liquid agent is contained in a packaging bag, it has strength enough to be taken out. The wet tissue package is encased in a container, and in the use of the wet tissues, they can be taken out one by one. Since the wet tissue exhibits water decomposability when contacted with a large amount of water, it can be disposed of by flushing it with flush toilet.

### EXAMPLE

The nonwoven fabric of the invention and a preferred embodiment of the body fluid absorber using the nonwoven fabric are further described with reference to the following examples, but it should be construed that the invention is in no way limited to those examples.

### Example 1

Fibers were prepared using a resin composition consisting of 70 % by weight of viscose rayon, 15 % by weight of sodium polyacrylate and 15 % by weight of cationized cellulose.

The process for the preparation of the fibers is described below.

To 70 parts of viscose prepared in a conventional manner and having a cellulose concentration of 9.0 % by weight and an alkali concentration of 5.7 % by weight, 30 parts of a mixed solution of sodium polyacrylate and cationized cellulose (sodium polyacrylate: 4.5 % by weight) was added, and they were sufficiently mixed by a stirrer to give a dispersion. Then, the dispersion was subjected to usual spinning to prepare fibers of 3.0 denier and 38 mm in cut length. In the spinning process, elution of a water-soluble polymer was hardly detected. The fibers were subjected to desulfurizing, rinsing with water, bleaching and water rinsing by a scouring machine. Then, the fibers were given a lubricant and dried.

The resulting fibers were measured on the properties (strength and elongation) in a dry state and a wet state (change of pH). The results are set forth in Table 1.

**Table 1**

| | | | |
|---|---|---|---|
| Dry strength (g/d) | Wet strength pH 6 (g/d) | Wet strength pH 9 (g/d) | Wet strength pH 10 (g/d) |
| 2.12 | 1.02 | 0.88 | 0.80 |
| Dry elongation (%) | Wet elongation pH 6 (%) | Wet elongation pH 9 (%) | Wet elongation pH 10 (%) |
| 14.5 | 29.6 | 28.0 | 21.5 |

### Example 2

From the fibers prepared in Example 1, a web was fabricated by using a test card. In this process, the web was entangled by a jet flow of 50 kg/cm² while moving the web at a rate of 7 m/min. The thus entangled web was dried to prepare a nonwoven fabric having METSUKE of 40 g/cm² and a thickness of 0.35 mm.

From the nonwoven fabric, a specimen having a width of 50 mm was obtained. The specimen was immersed in distilled water (pH value: about 5.5) for 10 minutes, and then elongated in the lengthwise direction and the crosswise direction under the conditions of a chuck distance of 100 mm and a tensile rate of 300 mm/min to measure the maximum load in each direction, which was taken as a tensile strength in each direction.

Separately, a specimen having a width of 50 mm was prepared, and the specimen was immersed in a 0.1 M disodium hydrogenphosphate solution (pH value: about 9.0) for 10 minutes. Then, the maximum load was measured under the same conditions as described above to obtain tensile strengths in the lengthwise and the crosswise directions.

The results are set forth in Table 2.

Further, 100 ml of distilled water (pH value: about 5.5) was placed in a 200 ml beaker, and a specimen of 2 cm x 2 cm having been cut from the nonwoven fabric was then placed in the water, followed by stirring with a magnetic stirrer at a constant rotational speed. Whether the nonwoven fabric was decomposed into fibers in water in the beaker and whether the fibers of the fabric were dispersed therein were observed. As a result, the nonwoven fabric exhibited water decomposability.

Separately, 100 ml of a 0.01 M disodium hydrogenphosphate solution (pH value: about 9.0) was placed in a 200 ml beaker, and a specimen of 2 cm x 2 cm having been cut from the nonwoven fabric in the same manner as above was then placed in the solution, followed by stirring with a magnetic stirrer at a constant rotational speed for 2 minutes. Whether the nonwoven fabric was decomposed into fibers in the solution in the beaker and whether the fibers of the fabric were dispersed therein were observed. As a result, the nonwoven fabric exhibited water decomposability after 30 seconds.

**Table 2**

| | | Dry tensile strength (kgf/50mm) | Tensile strength after immersion in distilled water (kgf/50mm) | Tensile strength after immersion in alkali water (kgf/50mm) |
|---|---|---|---|---|
| Example 2 | Lengthwise direction | 3.19 | 2.78 | 2.48 |
| | Crosswise direction | 1.07 | 0.84 | 0.61 |

### Example 3

Fibers were prepared using a resin composition consisting of 70 % by weight of viscose rayon, 15 % by weight of sodium polyacrylate and 15 % by weight of cationized cellulose.

The process for the preparation of the fibers is described below.

To 70 parts by weight of viscose prepared in a conventional manner and having a cellulose concentration of 9.0 % by weight of cellulose and an alkali concentration of 5.7 % by weight, 30 parts by weight of a mixed solution of sodium polyacrylate and cationized cellulose (sodium polyacrylate: 4.5 % by weight, cationized cellulose: 4.5 % by weight, sodium hydroxide: 0.5 % by weight) was added, and they were sufficiently mixed by a stirrer to give a dispersion. Then, the dispersion was subjected to usual spinning to prepare fibers of 3.0 denier and 38 mm in cut length. In the spinning process, elution of a water-soluble polymer was hardly detected. The fibers were subjected to desulfurizing, rinsing, bleaching and rinsing by a scouring machine. Then, the fibers were given a lubricant and dried.

From the fibers, a web was fabricated by using a test card. Then, the web was entangled by a highpressure water jet flow of 25 kg/cm² while moving the web at a rate of 6 m/min. The thus entangled web was dried to prepare a nonwoven fabric having METSUKE of 25 g/cm² and a thickness of 0.26 mm.

From the nonwoven fabric, a specimen having a width of 50 mm was obtained. The specimen was immersed in distilled water (pH value: about 5.5) for 10 minutes, and then elongated in the lengthwise direction and the crosswise direction under the conditions of a chuck distance of 100 mm and a tensile rate of 300 mm/min to measure the maximum load in each direction, which was taken as a tensile strength in each direction.

Separately, a specimen having a width of 50 mm was prepared, and the specimen was immersed in a 0.1 M disodium hydrogenphosphate solution (pH value: about 9.0) for 10 minutes. Then, the maximum load was measured under the same conditions as above to obtain tensile strengths in the lengthwise and the crosswise directions.

The results are set forth in Table 3.

Further, 100 ml of distilled water (pH value: about 5.5) was placed in a 200 ml beaker, and a specimen of 2 cm x 2 cm having been cut from the nonwoven fabric was then placed in the water, followed by stirring with a magnetic stirrer at a constant rotational speed. Whether the nonwoven fabric was decomposed into fibers in water in the beaker and whether the fibers of the fabric were dispersed therein were observed. As a result, the nonwoven fabric exhibited water decomposability after 2 seconds.

Separately, 100 ml of a 0.01 M disodium hydrogenphosphate solution (pH value: about 9.0) was placed in a 200 ml beaker, and a specimen of 2 cm x 2 cm having been cut from the nonwoven fabric in the same manner as above was then placed in the solution, followed by stirring with a magnetic stirrer at a constant rotational speed. Whether the nonwoven fabric was decomposed into fibers in the solution in the beaker and whether the fibers of the fabric were dispersed therein were observed. As a result, the nonwoven fabric exhibited water decomposability after 30 seconds.

### Example 4

Properties of the nonwoven fabric were measured in the same manner as in Example 1, except that the resin composition was replaced with a resin composition consisting of 50 % by weight of viscose rayon, 25 % by weight of sodium polyacrylate and 25 % by weight of cationized cellulose. The results are set forth in Table 3.

The nonwoven fabric exhibited water decomposability after 2 minutes in case of distilled water and after 30 seconds in case of alkali water, similarly to Example 1.

**Table 3**

| | | Dry tensile strength (kgf/50mm) | Tensile strength after immersion in distilled water (kgf/50mm) | Tensile strength after immersion in alkali water (kgf/50mm) |
|---|---|---|---|---|
| Example 3 | Lengthwise direction | 0.22 | - | - |
| | Crosswise direction | 0.05 | - | - |
| Example 4 | Lengthwise direction | 4.65 | 1.16 | 0.38 |
| | Crosswise direction | 1.06 | 0.13 | - |

### Example 5

A nonwoven fabric was prepared in the same manner as in Example 3, except that a mixture of 30 parts by weight of the fibers obtained in Example 3 and 70 parts by weight of pulp (average fiber length: 10 mm) was used.

The resulting nonwoven fabric was measured on the water decomposability in the same manner as in Example 3. The time required for decomposition of the nonwoven fabric in distilled water was 2 minutes, and the time required for decomposition thereof in a disodium hydrogenphosphate aqueous solution was 30 seconds. The tensile strength of the nonwoven fabric in a dry state and the tensile strength thereof after immersion in distilled water were measured in the same manner as in Example 3. The results are set forth in Table 4.

**Table 4**

| | | Dry tensile strength (kgf/50mm) | Tensile strength after immersion in distilled water (kgf/50mm) |
|---|---|---|---|
| Example 6 | Lengthwise direction | 2.40 | 0.90 |
| | Crosswise direction | 1.10 | 0.57 |

### Example 6

On one surface of the nonwoven fabric prepared in Example 5, a water-impermeable back sheet layer was formed, whereby a body fluid absorber was prepared. The body fluid absorber had excellent water absorption properties. After the absorption of water, the back sheet was separated from the body fluid absorber, and thereby the nonwoven fabric could be decomposed in water.

### Example 7

70 leaves of nonwoven fabrics (84 g) prepared in Example 5 were each folded and superposed on another so as to have an overlapped portion with the another, and they were encased in a water-impermeable packaging bag formed from a plastic laminated film having an aluminum evaporated layer. Into the packaging bag, 218 g of a liquid agent consisting of 97 parts by weight of water and 0.6 part by weight of sodium benzoate was introduced, and the open end was heat sealed to prepare a wet tissue package.

On the upper surface of the packaging bag of the wet tissue package, a cut to take out the encased wet tissues was formed, and at the cut area, a sealing member was bonded to the bag with an acrylic adhesive so as to cover the cut.

After the wet tissue package was allowed to stand for 90 days, the sealing member was peeled off, and the wet tissue package was encased in a rigid plastic container. The container was then lidded, and the wet tissues, which had been encased in the container together with the packaging bag, were taken out of the container one by one from the opening section formed on the top of the lid member. As a result, all the fabrics could be taken out without tearing.

The nonwoven fabrics thus taken out had water decomposability of almost the same level as that shown in Example 5.

## Claims

1. A nonwoven fabric comprising fibers of a resin composition comprising a cationic resin in an amount of 1 to 80% by weight and an anionic resin in an amount of 1 to 80% by weight.

2. The nonwoven fabric as claimed in claim 1, wherein the fibers are those formed from a resin composition comprising a regenerated cellulose in an amount of 20 to 98% by weight, a cationic resin in an amount of 1 to 79% by weight, and an anionic resin in an amount of 1 to 79% by weight.

3. The nonwoven fabric as claimed in claim 2, which comprises:
fibers of the resin composition comprising a regenerated cellulose, the cationic resin and the anionic resin, and
water-indecomposable short fibers.

4. The nonwoven fabric as claimed in claim 3, wherein said water-indecomposable short fibers are regenerated cellulose fibers having an average fiber length of less than 80 mm.

5. The nonwoven fabric as claimed in any one of claims 2 to 4, wherein said base resin or regenerated cellulose is at least one resin selected from the group consisting of viscose rayon, polynosic rayon, cupra and saponified acetate.

6. The nonwoven fabric as claimed in any one of claims 1 to 5, wherein said cationic resin is at least one resin selected from the group consisting of cationized cellulose, cationized starch, cationized cyamoposis gum, cationized dextrin and poly(dimethylmethylenepiperidinium chloride).

7. The nonwoven fabric as claimed in any one of claims 1 to 6, wherein said anionic resin is at least one resin selected from the group consisting of polyacrylic acid salt, carboxymethyl cellulose, carboxymethyl starch, alginic acid, xanthane gum and polymethyacrylic acid salt.

8. The nonwoven fabric as claimed in any one of claims 1 to 4, which has water decomposability.

9. A body fluid absorber having an absorbing layer comprising at least one nonwoven fabric selected from the group consisting of:
(a) a nonwoven fabric according to claim 1 comprising fibers formed from a resin composition comprising a cationic resin and an anionic resin,
(b) a nonwoven fabric according to claim 2 comprising fibers formed from a resin composition comprising a regenerated cellulose, a cationic resin and an anionic resin, and
(c) a nonwoven fabric according to claim 3 comprising fibers formed from a resin composition comprising a regenerated cellulose, a cationic resin and an anionic resin, and water-indecomposable short fibers.

10. A wet tissue comprising:
at least one nonwoven fabric selected from the group consisting of:
(a) a nonwoven fabric according to claim 1 comprising fibers formed from a resin composition comprising a cationic resin and an anionic resin,
(b) a nonwoven fabric according to claim 2 comprising fibers formed from a resin composition comprising a regenerated cellulose, a cationic resin and an anionic resin, and
(c) a nonwoven fabric according to claim 3 comprising fibers formed from a resin composition comprising a regenerated cellulose, a cationic resin and an anionic resin, and water-indecomposable short fibers; and
a liquid agent with which said nonwoven fabric is impregnated.

## Patentansprüche

1. Vliesstoff mit Fasern aus einer Harzzusammensetzung mit einem kationischen Harz in einer Menge von 1 bis 80 Gew.% und einem anionischen Harz in einer Menge von 1 bis 80 Gew.%.

2. Vliesstoff wie in Anspruch 1 beansprucht, wobei die Fasern jene sind, die aus einer Harzzusammensetzung mit einer regenerierten Zellulose in einer Menge von 20 bis 98 Gew.%, einem kationischen Harz in einer Menge von 1 bis 79 Gew.% und einem anionischen Harz in einer Menge von 1 bis 79 Gew.% gebildet sind.

3. Vliesstoff wie in Anspruch 2 beansprucht, der umfasst:
Fasern aus der Harzzusammensetzung mit einer regenerierten Zellulose, dem kationischen Harz und dem anionischen Harz, und wasserunzersetzbare Kurzfasern.

4. Vliesstoff wie in Anspruch 3 beansprucht, wobei die wasserunzersetzbaren Kuzenfasern regenerierte Zellulosefasern, mit einer mittleren Faserlänge von weniger als 80 mm sind.

5. Vliesstoff wie in irgendeinem der Ansprüche 2 bis 4 beansprucht, wobei das Basisharz oder die regenerierte Zellulose zumindest ein Harz ausgewählt aus der Gruppe, bestehend aus Viskose-Rayon, Polynosic-Rayon, Kupferseide und verseiftem Acetat, ist.

6. Vliesstoff wie in irgendeinem der Ansprüche 1 bis 5 beansprucht, wobei das kationische Harz zumindest eines ausgewählt aus der Gruppe ist, die besteht aus kationisierter Zellulose, kationisierter Stärke, kationisiertem Guargummi, kationisiertem Dextrin und Poly (Dimethylmethylenpiperidinchlorid).

7. Vliesstoff wie in irgendeinem der Ansprüche 1 bis 6 beansprucht, wobei das anionische Harz mindestens ein Harz ausgewählt aus der Gruppe ist, die besteht aus Polyacrylsäuresalz, Carboxymethylzellulose, Carboxymethylstärke, Alginsäure, Xanthangummi und Polymethylacrylsäuresalz.

8. Vliesstoff wie in irgendeinem der Ansprüche 1 bis 4 beansprucht, der Wasserzersetzbarkeit hat.

9. Körperflüssigkeitsabsorptionsmittel mit einer absorbierenden Schicht, das zumindest einen Vliesstoff enthält, ausgewählt aus der Gruppe, die besteht aus:
(a) einem Vliesstoff gemäß Anspruch 1 mit Fasern, die aus einer Harzzusammensetzung mit kationischem Harz und anionischem Harz gebildet sind,
(b) einem Vliesstoff gemäß Anspruch 2 mit Fasern, die aus einer Harzzusammensetzung mit regenerierter Zellulose, einem kationisichen Harz und einem anionischen Harz, gebildet sind, und
(c) einem Vliesstoff gemäß Anspruch 3 mit Fasern, die aus einer Harzzusammensetzung mit regenerierter Zellulose, einem kationisichen Harz und einem anionischen Harz und wasserunzersetzbaren Kurzfasern gebildet sind.

10. Feuchttuch enthaltend:
zumindest einen Vliesstoff ausgewählt aus der Gruppe, bestehend aus:
(a) einem Vliesstoff gemäß Anspruch 1 mit Fasern, die aus einer Harzzusammensetzung mit einem kationisichen Harz und einem anionischen Harz gebildet sind,
(b) einem Vliesstoff gemäß Anspruch 2 mit Fasern, die aus einer Harzzusammensetzung mit regenerierter Zellulose, einem kationisichen Harz und einem anionischen Harz gebildet sind und
(c) einem Vliesstoff gemäß Anspruch 3 mit Fasern, die aus einer Harzzusammensetzung mit regenerierter Zellulose, einem kationisichen Harz und einem anionischen Harz, und wasserunzersetzbaren Kurzfasern gebildet sind; und
ein flüssiges Mittel mit dem der Vliesstoff imprägniert ist.

## Revendications

1. Tissu non tissé comprenant des fibres faites d'une composition de résine comprenant une résine cationique dans une proportion de 1 à 80 % en poids et une résine anionique dans une proportion de 1 à 80 % en poids.

2. Tissu non tissé tel que revendiqué dans la revendication 1, dans lequel les fibres sont formées à partir d'une composition de résine comprenant de la cellulose régénérée dans une proportion de 20 à 98 % en poids, une résine cationique dans une proportion de 1 à 79 % en poids, et une résine anionique dans une proportion de 1 à 79 % en poids.

3. Tissu non tissé tel que revendiqué dans la revendication 2, lequel comprend :
- des fibres faites de la composition de résine comprenant de la cellulose régénérée, la résine cationique et la résine anionique ; et
- des fibres courtes non décomposables dans l'eau.

4. Tissu non tissé tel que revendiqué dans la revendication 3, dans lequel lesdites fibres courtes non décomposables dans l'eau consistent en des fibres de cellulose régénérée ayant une longueur de fibre moyenne de moins de 80 mm.

5. Tissu non tissé tel que revendiqué dans l'une quelconque des revendications 2 à 4, dans lequel ladite résine de base ou ladite cellulose régénérée consistent en au moins une résine choisie dans le groupe consistant en de la rayonne viscose, de la rayonne polynosique, du cupra et de l'acétate saponifié.

6. Tissu non tissé tel que revendiqué dans l'une quelconque des revendications 1 à 5, dans lequel ladite résine cationique consiste en au moins une résine choisie dans le groupe consistant en de la cellulose cationisée, de l'amidon cationisé, de la gomme de cyamoposis cationisée, de la dextrine cationisée et du polychlorure de diméthylméthylènepipéridinium.

7. Tissu non tissé tel que revendiqué dans l'une quelconque des revendications 1 à 6, dans lequel ladite résine anionique consiste en au moins une résine choisie dans le groupe consistant en du sel d'acide polyacrylique, de la carboxyméthylcellulose, de l'amidon carboxyméthylé, de l'acide alginique, de la gomme de xanthane et du sel d'acide polyméthacrylique.

8. Tissu non tissé tel que revendiqué dans l'une quelconque des revendications 1 à 4, lequel est décomposable dans l'eau.

9. Absorbeur de fluide corporel comportant une couche absorbante comprenant au moins un tissu non tissé choisi dans le groupe consistant en :
(a) un tissu non tissé selon la revendication 1, comprenant des fibres formées à partir d'une composition de résine comprenant une résine cationique et une résine anionique ;
(b) un tissu non tissé selon la revendication 2, comprenant des fibres formées à partir d'une composition de résine comprenant de la cellulose régénérée, une résine cationique et une résine anionique ; et
(c) un tissu non tissé selon la revendication 3, comprenant des fibres formées à partir d'une composition de résine comprenant de la cellulose régénérée, une résine cationique et une résine anionique, ainsi que des fibres courtes non décomposables dans l'eau.

10. Tissu humide comprenant au moins un tissu non tissé choisi dans le groupe consistant en :
(a) un tissu non tissé selon la revendication 1, comprenant des fibres formées à partir d'une composition de résine comprenant une résine cationique et une résine anionique ;
(b) un tissu non tissé selon la revendication 2, comprenant des fibres formées à partir d'une composition de résine comprenant de la cellulose régénérée, une résine cationique et une résine anionique ; et
(c) un tissu non tissé selon la revendication 3, comprenant des fibres formées à partir d'une composition de résine comprenant de la cellulose régénérée, une résine cationique et une résine anionique, ainsi que des fibres courtes non décomposables dans l'eau ; et
un agent liquide dont ledit tissu non tissé est imprégné.
